(19) Europäisches Patentamt
European Patent Office
Office européen des brevets

(11) Veröffentlichungsnummer: **0 491 968 B1**

(12) **EUROPÄISCHE PATENTSCHRIFT**

(45) Veröffentlichungstag der Patentschrift: **26.10.94**

(51) Int. Cl.$^5$: **C07C 209/26**, C07C 211/48

(21) Anmeldenummer: **90124846.8**

(22) Anmeldetag: **20.12.90**

(54) **Verfahren zur Herstellung von N-Alkyl-fluoranilinen.**

(30) Priorität: **02.02.90 DE 4003078**

(43) Veröffentlichungstag der Anmeldung:
**01.07.92 Patentblatt 92/27**

(45) Bekanntmachung des Hinweises auf die Patenterteilung:
**26.10.94 Patentblatt 94/43**

(84) Benannte Vertragsstaaten:
**BE CH DE FR GB IT LI NL**

(56) Entgegenhaltungen:
**WO-A-91/00262**
**DE-A- 2 941 070**
**DE-B- 1 014 547**
**DE-B- 1 249 279**
**DE-C- 929 806**
**US-A- 3 261 869**
**US-A- 3 509 213**

**JOURNAL OF THE AMERICAN CHEMICAL SOCIETY, 20. Juni 1965, Seiten 2767-2768, Washington, DC, US; F.S. DOVELL et al.: "Platinum metal sulfides as heteregeneous hydrogenation catalysts"**

(73) Patentinhaber: **BAYER AG**

**D-51368 Leverkusen (DE)**

(72) Erfinder: **Maurer, Fritz, Dr., Nihon Tokushu Noyaku Seizo K.K.,**
**Yuki Research Center**
**9511-4 Yuki, Yuki-shi, Ibaraki 307 (JP)**
Erfinder: **Rohe, Lothar, Dr.**
**Damaschkeweg 75**
**W-5600 Wuppertal 1 (DE)**
Erfinder: **Knops, Hans-Joachim, Dr.**
**Köpenicker Strasse 35**
**W-4019 Monheim (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

## Beschreibung

Die Erfindung betrifft ein neues Verfahren zur Herstellung von N-Alkyl-fluoranilinen, welche als Zwischenprodukte für landwirtschaftlich nutzbare Stoffe, insbesondere für Herbizide, verwendet werden können.

Es ist bekannt, daß man N-Alkyl-arylamine erhält, wenn man Arylamine entweder mit Alkylhalogeniden (vgl. J. Org. Chem. 24 (1959), S. 1551-1553) oder mit Ketonen in Gegenwart von Natriumborhydrid und Essigsäure (vgl. J. Am. Chem. Soc. 96 (1974), S. 7812-7814; US-P 4 418 021) umsetzt.

Grundsätzlich bekannt ist weiterhin, daß N-Alkyl-arylamine durch reduktive Alkylierung aus aromatischen Nitroverbindungen mittels katalytischer Hydrierung in Gegenwart von aliphatischen Ketonen hergestellt werden können (vgl. "Organic Reactions", Coll. Vol. 4, John Wiley and Sons, Inc., New York, 1948, S. 174 ff, bes. S. 188-189; J. Org. Chem. 29 (1964), S. 1265; US-P 3 522 309).

Ausbeute und/oder Qualität der so hergestellten Produkte sind jedoch in vielen Fällen unbefriedigend.

Aus den nachfolgend genannten Dokumenten sind weitere Beispiele für die Herstellung von N-Alkyl-arylaminen aus den entsprechenden aromatischen Nitroverbindungen durch katalytische Hydrierung in Gegenwart von entsprechenden Carbonylverbindungen (d.h. nach der generellen Methode der reduktiven Alkylierung) bekannt. So beschreiben die Dokumente DE-B-1 014 547, DE-C-929 806, DE-B-1 249 279, US-A-3 509 213 und US-A-3 261 869 die Herstellung von verschiedenen, nicht kernhalogenierten N-Alkyl-arylaminen, wobei übliche, normal aktive - nicht desaktivierte - Hydrierungskatalysatoren (z.B. mit einem Gehalt an Platin, Palladium, Cobalt, Rhenium oder Nickel) eingesetzt werden.

Die Dokumente DE-A-2 941 070, J.Amer.Chem.Soc. 87:12 (1965), Seiten 2767-2768, sowie EP-A-0 479 877 (= WO 91/00262) beschreiben die Herstellung von kernchlorierten und kernbromierten N-Alkyl-arylaminen nach der gleichen Methode; jedoch werden in diesen Fällen speziell desaktivierte bzw. (im Vergleich zu den sonst üblichen, normal aktiven Pd- oder Pt-Katalysatoren) relativ inaktive Hydrierungskatalysatoren verwendet, nämlich entweder ein speziell sulfitierter, und dadurch desaktivierter, Platin-auf-Kohlenstoff-Katalysator (hergestellt gemäß DE-A-2 105 780) oder verschiedene Platinmetall-Sulfid-Katalysatoren, d.h. mit einem Gehalt an Ruthenium-, Rhodium-, Palladium-, Osmium-, Iridium- und/oder Platin-Sulfid. Durch Verwendung von solchen desaktivierten Katalysatoren wird eine unerwünschte Enthalogenierung im Reaktionsprodukt verhindert.

Über die Herstellung von kernfluorierten N-Alkyl-arylaminen, wie z.B. N-Akyl-fluoranilinen, nach der gleichen Grundmethode der reduktiven Alkylierung ist aus dem Stand der Technik nichts bekannt.

Es wurde nun überraschend gefunden, daß man N-Alkyl-fluoraniline der allgemeinen Formel (I)

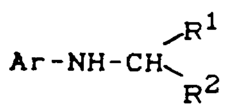

(I)

in welcher

- Ar für einen Phenylrest steht, welcher einfach oder zweifach durch Fluor substituiert ist,
- $R^1$ für Wasserstoff, Methyl, Ethyl, Propyl, Isopropyl, Butyl oder Isobutyl steht und
- $R^2$ für Methyl, Ethyl, Propyl, Isopropyl, Butyl, Isobutyl, sec-Butyl, tert-Butyl oder Phenyl steht oder zusammen mit $R^1$ für Ethan-1,2-diyl, Propan-1,3-diyl, Butan-1,4-diyl oder Pentan-1,5-diyl steht,

in sehr guten Ausbeuten und in hoher Reinheit erhält, wenn man in einem "Ein-Topf-Verfahren" Fluornitrobenzole der allgemeinen Formel (II)

$Ar\text{-}NO_2$ (II)

in welcher

- Ar die oben angegebene Bedeutung hat,

mit Carbonylverbindungen der allgemeinen Formel (III)

$R^1\text{-}CO\text{-}R^2$ (III)

in welcher

- $R^1$ und $R^2$ die oben angegebene Bedeutung haben,

in Gegenwart von Wasserstoff und in Gegenwart eines Palladium- oder Platin-Katalysators - bestehend aus Palladium-Metall, Palladium(II)-acetat, Palladium(II)-chlorid, Palladium(II)-nitrat, Palladium(II)-oxid, Platin-Metall, Platin(II)-chlorid, Platin(IV)-chlorid, oder Platin(IV)-oxid, wobei die Metallsalze gegebenenfalls als Hydrate eingesetzt werden können - bei Temperaturen zwischen 0 °C und 200 °C, gegebenenfalls bei erhöhtem Druck, umsetzt.

Verwendet man beispielsweise 4-Fluor-nitrobenzol und Aceton als Ausgangsstoffe, so kann der Reaktionsablauf durch das folgende Formelschema skizziert werden:

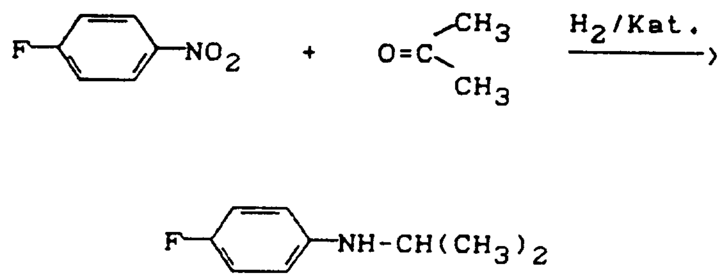


Die als Ausgangsstoffe zu verwendenden Fluor-nitrobenzole sind durch die Formel (II) allgemein definiert.

Als Beispiele für die Ausgangsstoffe der Formel (II) seien genannt:
2-Fluor-, 3-Fluor-, 4-Fluor-, 2,3-Difluor-, 2,4-Difluor-, 2,5-Difluor-, 2,6-Difluor-, 3,4-Difluor- und 3,5-Difluornitrobenzol.

Die Ausgangsstoffe der Formel (II) sind bekannt und/oder können nach an sich bekannten Verfahren hergestellt werden (vgl. z.B. J. Am. Chem. Soc. 72 (1950), 2296-2297).

Die weiter als Ausgangsstoffe zu verwendenden Carbonylverbindungen sind durch die Formel (III) allgemein definiert.

Als Beispiele für die Ausgangsstoffe der Formel (III) seien genannt:
Acetaldehyd, Propionaldehyd, Butyraldehyd, Isobutyraldehyd, Benzaldehyd, Aceton, Methylethylketon, Methylpropylketon, Methylisopropylketon, Methylbutylketon, Methylisobutylketon, Methyl-sec-butylketon, Methyl-tert-butylketon, Diethylketon, Dipropylketon, Diisopropylketon, Diisobutylketon, Acetophenon, Cyclopropanon, Cyclobutanon, Cyclopentanon und Cyclohexanon.

Die Ausgangsstoffe der Formel (III) sind bekannte organische Synthesechemikalien.

Die erfindungsgemäß zu verwendenden Katalysatoren sind oben genannt worden.

Die Katalysatoren werden vorzugsweise fein verteilt auf geeigneten Trägermaterialien, wie z.B. Aktivkohle oder Kieselgur, eingesetzt. Als Beispiele für besonders geeignete Katalysator-Träger-Kombinationen seien Palladium oder Platin auf Aktivkohle genannt.

Das erfindungsgemäße Verfahren kann in Gegenwart von Verdünnungsmitteln durchgeführt werden. In Frage kommen aliphatische und aromatische Kohlenwasserstoffe wie z.B. Benzin, Benzol und Toluol, aliphatische Alkohole wie z.B. Methanol, Ethanol, Propanol und Isopropanol, ferner Ether wie z.B. Diethyl-, Diisopropyl-, Methyl-tert.-butyl- und Methyl-tert.-amylether, Tetrahydrofuran und Dioxan.

Die Reaktionstemperaturen können beim erfindungsgemäßen Verfahren in einem größeren Bereich variiert werden. Im allgemeinen arbeitet man bei Temperaturen zwischen 0 °C und 200 °C, vorzugsweise zwischen 20 °C und 160 °C, insbesondere zwischen 30 °C und 120 °C.

Das erfindungsgemäße Verfahren wird im allgemeinen im Druckbereich zwischen 1 und 200 bar, vorzugsweise zwischen 5 und 150 bar, insbesondere zwischen 10 und 120 bar durchgeführt.

Zur Durchführung des erfindungsgemäßen Verfahrens setzt man auf 1 Mol Fluor-nitrobenzol der Formel (II) im allgemeinen zwischen 1 und 15 Mol, insbesondere zwischen 1 und 10 Mol, Carbonylverbindung der Formel (III) ein.

Die Ausgangsstoffe der Formeln (II) und (III) werden - gegebenenfalls in einem Verdünnungsmittel und vorzugsweise in einem Autoklaven - vermischt und nach Zugabe eines Pd- oder Pt-Katalysators bis zum Ende der Wasserstoff-Aufnahme hydriert. Der Katalysator wird durch Filtrieren oder Zentrifugieren abgetrennt. Die verbleibende Lösung wird durch Destillation - vorzugsweise unter vermindertem Druck - aufgearbeitet.

Die nach dem erfindungsgemäßen Verfahren herzustellenden N-Alkyl-fluoraniline der Formel (I) können als Zwischenprodukte für landwirtschaftlich nutzbare Stoffe verwendet werden (vgl. US-P 4 418 021, DE-OS 38 21 600).

Herstellungsbeispiele:

Beispiel 1

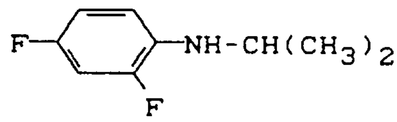

Eine Mischung aus 10,0 g (63 mMol) 2,4-Difluor-nitrobenzol, 50 ml Aceton und 0,5 g Palladium/Kohle-Katalysator (mit 5% Pd) wird bei 60 °C und 100 bar Wasserstoffdruck hydriert. Nach Ende der Wasserstoff-Aufnahme wird der Katalysator durch Absaugen abgetrennt und vom Filtrat wird das Lösungsmittel im Wasserstrahlvakuum bei 30 °C sorgfältig abdestilliert.

Man erhält 10,0 g (93% der Theorie) N-Isopropyl-2,4-difluor-anilin als amorphen Rückstand mit einem gaschromatographisch bestimmten Gehalt von 97,7%;

Retentions-Index RI (OV 101): 1092.

*

Bei Beispiel 1 und den folgenden Beispielen wird zur Charakterisierung der erhaltenen Produkte der gaschromatographisch ermittelte Retentions-Index RI (OV 101) angegeben, wobei sich die Angabe "OV 101" auf das verwendete Säulenmaterial bezieht, nämlich Dimethylsilicone der Firma "Chrompack" mit der Bezeichnung "DB1".

Zur exakten Definition des Retentions-Index vgl. Milton L. Lee, Frank J. Yang und Keith D. Bartle, "Open Tubular Column Gas Chromatography, Theory and Practice, John Wiley & Sons, New York (1984), S. 216 ff.

Referenzbeispiel (wobei ein nicht anspruchsgemäß einzusetzender Ausgangsstoff verwendet wird):

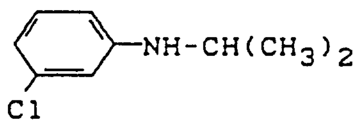

Eine Mischung aus 15,8 g (0,1 Mol) 3-Chlornitrobenzol, 50 ml Methanol, 8,5 ml Aceton und 0,3 g Platin/Kohle-Katalysator (5 % Pt) wird bei 60 °C und 50 bar Wasserstoffdruck hydriert. Nach Ende der Wasserstoffaufnahme wird der Katalysator durch Absaugen abgetrennt und vom Filtrat wird das Lösungsmittel im Wasserstrahlvakuum bei 30 °C sorgfältig abdestilliert.

Man erhält 16,1 g (95 % der Theorie) 3-Chlor-N-isopropyl-anilin als Flüssigkeit mit einem gaschromatographisch bestimmten Gehalt von 92,5 %; Retentions-Index* RI (OV 101): 1336.

Analog Beispiel 1 und Referenzbeispiel können beispielsweise auch die in der nachstehenden Tabelle 1 aufgeführten Verbindungen der Formel (I) hergestellt werden:

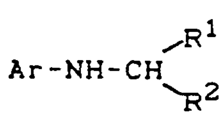

(I)

Tabelle 1:

| Bsp.-Nr. | Ar | $R^1$ | $R^2$ | Ausbeute (% d.Th.) | RI* (OV 101) |
|---|---|---|---|---|---|
| 2 | 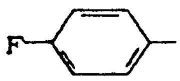 | $CH_3$ | $CH_3$ | 90,5 | 1146 |
| 3 | 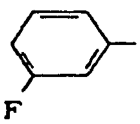 | $CH_3$ | $CH_3$ | 92 | 1155 |
| 4 | 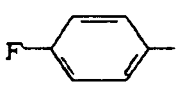 | $CH_3$ | $CH_2CH(CH_3)_2$ | 93 | 1385 |

Tabelle 1 - Fortsetzung

| Bsp.-Nr. | Ar | $R^1$ | $R^2$ | Ausbeute (% d.Th.) | RI* (OV 101) |
|---|---|---|---|---|---|
| 5 | 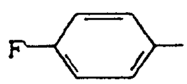 | $-CH_2CH_2CH_2CH_2-$ | | | |
| 6 | 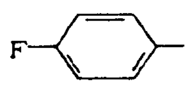 | H | $C_3H_7$ | | |
| 7 | 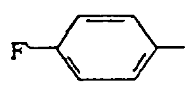 | $-CH_2CH_2CH_2CH_2CH_2-$ | | 95 | 1549 |
| 8 | 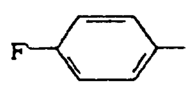 | $C_3H_7$ | $CH_3$ | 95 | 1332 |

## Patentansprüche

**1.** Verfahren zur Herstellung von N-Alkyl-fluoranilinen der allgemeinen Formel (I)

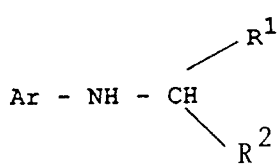
(I)

in welcher

Ar für einen Phenylrest steht, welcher einfach oder zweifach durch Fluor substituiert ist,

$R^1$ für Wasserstoff, Methyl, Ethyl, Propyl, Isopropyl, Butyl oder Isobutyl steht und

$R^2$ für Methyl, Ethyl, Propyl, Isopropyl, Butyl, Isobutyl, sec-Butyl, tert-Butyl oder Phenyl steht oder zusammen mit $R^1$ für Ethan-1,2-diyl, Propan-1,3-diyl, Butan-1,4-diyl oder Pentan-1,5-diyl steht,

dadurch gekennzeichnet, daß man Fluor-nitrobenzole der Formel (II)

$Ar\text{-}NO_2$ (II)

in welcher
Ar die oben angegebene Bedeutung hat,
mit Carbonylverbindungen der allgemeinen Formel (III)

$R^1\text{-}CO\text{-}R^2$ (III)

in welcher
$R^1$ und $R^2$ die oben angegebene Bedeutung haben,
in Gegenwart von Wasserstoff und in Gegenwart eines Palladium- oder Platin-Katalysators - bestehend aus Palladium-Metall, Palladium(II)-acetat, Palladium(II)-chlorid, Palladium(II)-nitrat, Palladium(II)-oxid, Platin-Metall, Platin(II)-chlorid, Platin(IV)-chlorid, oder Platin(IV)-oxid, wobei die Metallsalze gegebenenfalls als Hydrate eingesetzt werden können - bei Temperaturen zwischen 0°C und 200°C, gegebenenfalls bei erhöhtem Druck, umsetzt.

**2.** Verfahren nach Anspruch 1, dadurch gekennzeichnet, daß als Fluor-nitrobenzol (II) 4-Fluor-nitrobenzol und als Carbonylverbindung (III) Aceton eingesetzt werden.

**3.** Verfahren nach Anspruch 1, dadurch gekennzeichnet, daß man bei Temperaturen zwischen 20°C und 160°C arbeitet.

**4.** Verfahren nach Anspruch 1, dadurch gekennzeichnet, daß man bei Temperaturen zwischen 30°C und 120°C arbeitet.

**5.** Verfahren nach Anspruch 1, dadurch gekennzeichnet, daß man im Druckbereich von 1-200 bar, bevorzugt von 5-150 bar, besonders bevorzugt von 10-120 bar arbeitet.

**6.** Verfahren nach Anspruch 1, dadurch gekennzeichnet, daß man auf 1 Mol Fluor-nitrobenzol (II) 1-15 Mol, bevorzugt 1-10 Mol Carbonylverbindung (III) einsetzt.

**7.** Verfahren nach Anspruch 1, dadurch gekennzeichnet, daß man den Palladium- bzw. Platin-Katalysator in Form einer Katalysator-Träger-Kombination - fein verteilt auf Aktivkohle - einsetzt.

## Claims

**1.** Process for the preparation of N-alkyl-fluoroanilines of the general formula (I)

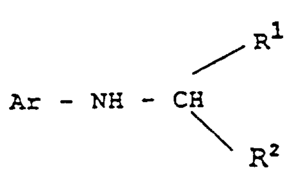

(I)

in which
Ar represents a phenyl radical which is monosubstituted or disubstituted by fluorine,
$R^1$ represents hydrogen, methyl, ethyl, propyl, isopropyl, butyl or isobutyl and
$R^2$ represents methyl, ethyl, propyl, isopropyl, butyl, isobutyl, sec-butyl, tert-butyl or phenyl, or, together with $R^1$, represents ethane-1,2-diyl, propane-1,3-diyl, butane-1,4-diyl or pentane-1,5-diyl,
characterized in that fluoro-nitrobenzenes of the formula (II)

$Ar\text{-}NO_2$ (II)

in which

Ar has the abovementioned meaning,

are reacted with carbonyl compounds of the general formula (III)

$R^1$-CO-$R^2$ (III)

in which

$R^1$ and $R^2$ have the abovementioned meaning,

in the presence of hydrogen and in the presence of a palladium or platinum catalyst - consisting of palladium metal, palladium(II) acetate, palladium(II) chloride, palladium(II) nitrate, palladium(II) oxide, platinum metal, platinum(II) chloride, platinum(IV) chloride or platinum(IV) oxide, it optionally being possible to employ the metal salts as hydrates - at temperatures between 0 °C and 200 °C, if appropriate at elevated pressure.

**2.** Process according to Claim 1, characterized in that 4-fluoro-nitrobenzene is employed as the fluoronitrobenzene (II) and acetone is employed as the carbonyl compound (III).

**3.** Process according to Claim 1, characterized in that the process is carried out at temperatures between 20 °C and 160 °C.

**4.** Process according to Claim 1, characterized in that the process is carried out at temperatures between 30 °C and 120 °C.

**5.** Process according to Claim 1, characterized in that the process is carried out in a pressure range of 1-200 bar, preferably of 5-150 bar, particularly preferably of 10-120 bar.

**6.** Process according to Claim 1, characterized in that 1-15 moles, preferably 1-10 moles, of carbonyl compound (III) are employed per mole of fluoronitrobenzene (II).

**7.** Process according to Claim 1, characterized in that the palladium or platinum catalyst is employed in the form of a catalyst/support combination - finely divided on activated charcoal.

**Revendications**

**1.** Procédé de production de N-alkyl-fluoranilines de formule générale (I)

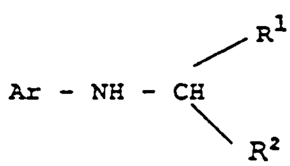

(I)

dans laquelle

Ar représente un radical phényle qui peut être substitué une ou deux fois par du fluor,

$R^1$ représente de l'hydrogène, un radical méthyle, éthyle, propyle, isopropyle, butyle ou isobutyle, et

$R^2$ représente un radical méthyle, éthyle, propyle, isopropyle, butyle, isobutyle, sec-butyle, tert-butyle ou phényle, ou représente avec $R^1$ un radical éthane-1,2-diyle, propane-1,3-diyle, butane-1,4-diyle ou pentane-1,5-diyle,

caractérisé en ce que l'on fait réagir des fluoronitrobenzènes de formule générale (II)

Ar-$NO_2$ (II)

dans laquelle Ar a la signification précitée,

avec des composés carbonyliques de formule générale (III)

$R^1$-CO-$R^2$ (III)

dans laquelle $R^1$ et $R^2$ ont les significations précitées, en présence d'hydrogène et en présence d'un catalyseur au palladium ou au platine, constitué de palladium métallique, d'acétate de palladium (II), de chlorure de palladium (II), de nitrate de palladium (II), d'oxyde de palladium (II), de platine métallique, de chlorure de platine (II), de chlorure de platine (IV) ou d'oxyde de platine (IV), les sels métalliques pouvant être utilisés éventuellement sous forme d'hydrates à des températures entre 0 °C et 200 °C, éventuellement sous pression élevée.

**2.** Procédé selon la revendication 1, caractérisé en ce que l'on utilise, comme fluoronitrobenzène (II), du 4-fluoronitrobenzène et, comme composé carbonylique (III), de l'acétone.

**3.** Procédé selon la revendication 1, caractérisé en ce que l'on opère à des températures comprises entre 20 °C et 160 °C.

**4.** Procédé selon la revendication 1, caractérisé en ce que l'on opère à des températures comprises entre 30 °C et 120 °C.

**5.** Procédé selon la revendication 1, caractérisé en ce que l'on opère dans un domaine de pression de 1 à 200 bar et, de préférence, de 5 à 150 bar et, plus particulièrement, de 10 à 120 bar.

**6.** Procédé selon la revendication 1, caractérisé en ce que l'on utilise, pour une mole de fluoronitrobenzène (II), de 1 à 15 moles et, de préférence, de 1 à 10 moles de composé carbonylique (III).

**7.** Procédé selon la revendication 1, caractérisé en ce que l'on utilise le catalyseur au palladium ou au platine sous forme d'une combinaison de catalyseur et de support finement divisé sur du charbon actif.